# EUROPEAN PATENT SPECIFICATION

(11) **EP 4 470 580 B1**
(45) Date of publication and mention of the grant of the patent: **10.09.2025**
(21) Application number: 24179222.5
(22) Date of filing: 31.05.2024
(51) Int. Cl.: A61M 5/14, A61M 25/02

(54) **MEDICAL-TUBE AFFIXING DEVICE**
VORRICHTUNG ZUR BEFESTIGUNG MEDIZINISCHER SCHLÄUCHE
DISPOSITIF DE FIXATION DE TUBE MÉDICAL

(30) Priority: 31.05.2023 TW 112120406
(43) Date of publication of application: 04.12.2024
(73) Proprietor: Archer Biotechnology Development Co., Ltd., Taichung City 404034 (TW); Su, Chien-Chung, Taichung City 404034 (TW)
(72) Inventor: SU, Chien-Chung, 404034 Taichung City (TW); HAN, Hsin-Liang, 404034 Taichung City (TW)
(74) Representative: Viering, Jentschura & Partner mbB Patent- und Rechtsanwälte

(56) References cited:
- CN-A- 113 384 467
- CN-U- 213 759 931
- US-A- 5 084 026

## Description

### BACKGROUND OF THE INVENTION

### 1. Technical Field

The present invention relates to a medical-tube affixing device, and more particularly to a medical-tube affixing device developed primarily for extracorporeal membrane oxygenation (ECMO) in medical applications.

### 2. Description of Related Art

Tubes and pipes are used in various medical treatments, and may be known as intravenous drips, cannulas, urinary catheters, oxygen hoses, thoracic ducts, nasogastric tubes, tracheostomy tubes, and so on. Some of these tubes are for long use and have to be well fixed to patients in order to prevent tubes from unintentionally loosing or falling or entangling with patients' limbs.

Similarly, some patients may have to use indwelling catheters for long periods due to their conditions. For example, for patients with long term mechanical ventilation, patients after surgical operations, and parents with some catastrophic illness, doctors may instruct them to intermittently suspend use of the tubes according to their conditions instead of removing the tubes. In such a rest period, it is necessary to properly fix the tubes. Otherwise, once the tubes are bended or compressed, or even coming off, the patients are subject to the risk of infection and the pain caused by reinstallation of tubes. Traditionally, healthcare workers fix these tubes with pins, adhesive tape, and securement devices. Fixation with pins is less firm and tends to fail due to looseness or slippage. Adhesive tape can be ineffective in fixation when tied too loose and can cause pressure ulcers when tied too tight. Securement devices are hard to apply and are not reusable so they have to be entirely replaced when wound care, dressing change or repositioning is required. Therefore, the traditional affixing means are unsatisfying in terms of operational convenience and fixation performance.

As attempts to solve the problems, some medical-tube affixing devices have been developed. According to existing technical literature, some example of the known medical-tube affixing devices are China Patent Publication No. CN218589449U titled "Kidney internal medicine deep vein temporary hemodialysis catheter fixing device," China Patent Publication No. CN216963267U titled "Portable medical catheter fixing clamp," Taiwan Patent No. TWI747549B titled "Nasogastric tube fixing device" that have been granted to the inventor of the present invention, China Patent Publication No. CN213347378U titled "Oxygen tube capable of preventing skin from being damaged and gastric catheter fixing frame," Taiwan Patent Publication No. TWM546227U titled "Securing and receiving device of gastrostomy tube," China Patent Publication No. CN218793557U titled "Closed drainage tube clamping device for thoracic surgery" among others. However, none of these prior-art devices can fix multiple tubes while providing convenience in adjusting the device to fit tubes of different sizes and in repositioning the device, and thus none of these prior-art devices can effectively improving working efficiency of healthcare workers.

### SUMMARY OF THE INVENTION

To address the shortcomings of the existing medical-tube affixing devices, the inventor has conducted extensive research and experiments and finally achieve substantive improvements over the prior art.

The present invention provides a medical-tube affixing device, which comprises: an adhesive member having one side adhering to a patient; a base, being fixed to a reverse side of the adhesive member; and a clipping assembly, being movably mounted on the base and being configured to be operated to hold a plurality of medical tubes installed on the body of the patient.

In another embodiment of the present invention, the adhesive member adheres to the patient at one side thereof, and the base has a U-shaped body that is fixed to a reverse side of the adhesive member.

In another embodiment of the present invention, the base defines a slide space formed as a dovetail mortise, two retaining portions that are slender and raised, and two first pivot holes, wherein the two retaining portions are located at a bottom of the slide space, and the first pivot holes are located at two diagonally opposite free corners of the base remote from the retaining portions, in which a first slider is shaped as a dovetail tenon matching the dovetail-mortise-shaped slide space of the base.

In another embodiment of the present invention, a first movable fastener is U-shaped.

In another embodiment of the present invention, a first curved arm and a second curved arm each have a flexible, C-shaped body, and a channel is formed as a through hole with a trapezoid cross-section.

In another embodiment of the present invention, a second slider has a trapezoid body.

In another embodiment of the present invention, the channel of the first slider has two stopping portions in the channel, wherein the stopping portion are configured to engage with two stoppers raised from a bottom of the second slider.

The primary objective of the present invention, a medical-tube affixing device, is to enable a healthcare worker to attach or detach multiple medical tubes to or from a patient easily. The medical-tube affixing device can be adjusted to fit the medical tubes of different sizes and can be repositioned easily, thereby improving working efficiency.

### BRIEF DESCRIPTION OF THE DRAWINGS

**FIG. 1** is a perspective view of the present invention.
**FIG. 2** is an exploded of the present invention.
**FIG. 3** is a cross-sectional, operational view of the present invention.
**FIG. 4** is another cross-sectional, operational view of the present invention.
**FIG. 5** is a further cross-sectional, operational view of the present invention.
**FIG. 6** is an applied view of the present invention.

### DETAILED DESCRIPTION OF THE INVENTION

For further illustrating the means and functions by which the present invention achieves the certain objectives, the following description, in conjunction with the accompanying drawings and preferred embodiments, is set forth as below to illustrate the implement, structure, features and effects of the subject matter of the present invention.

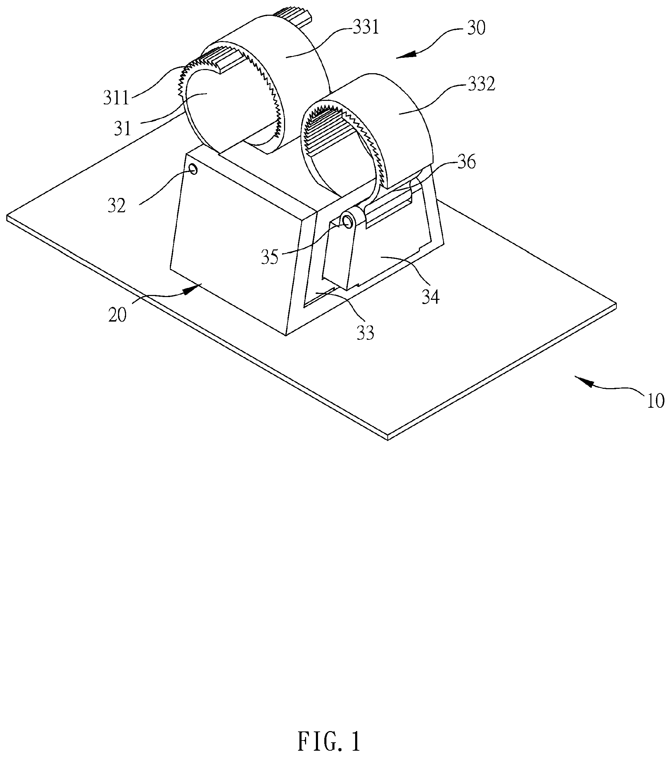

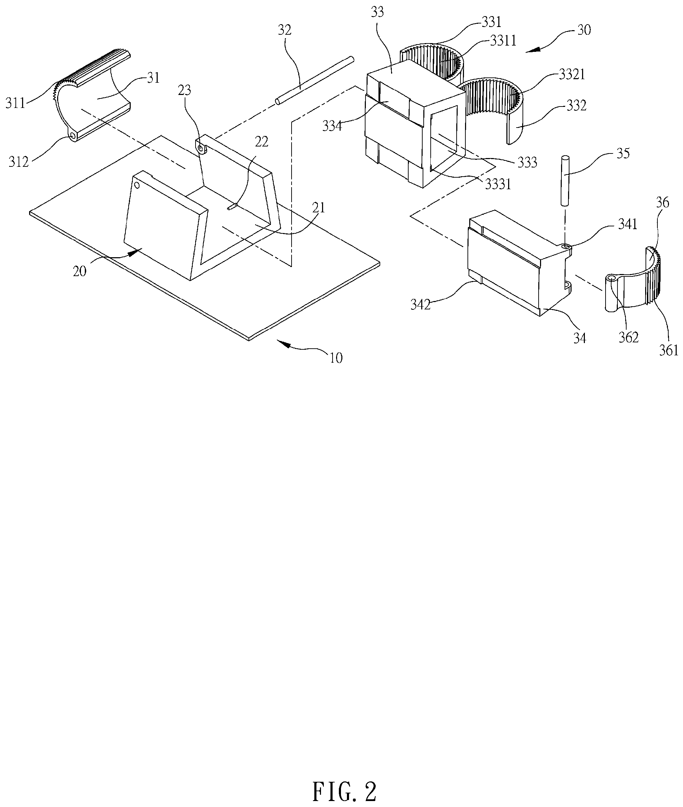

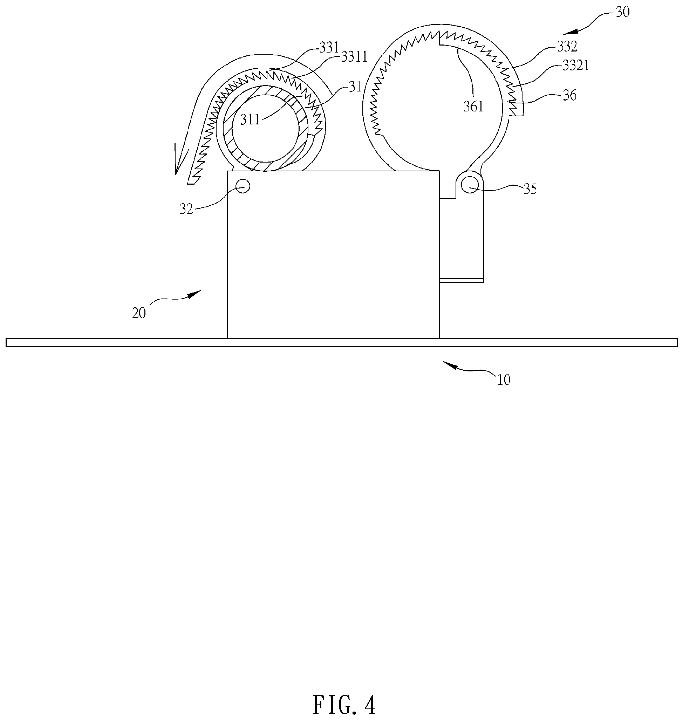

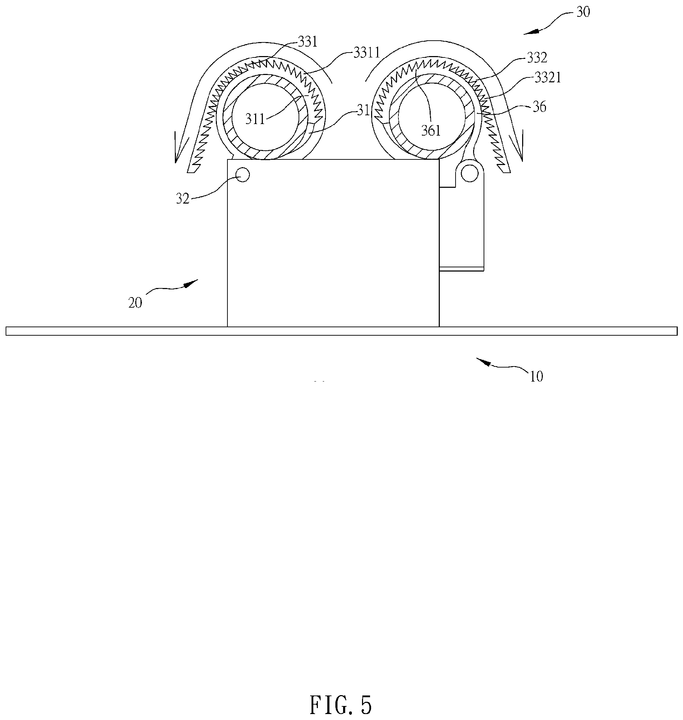

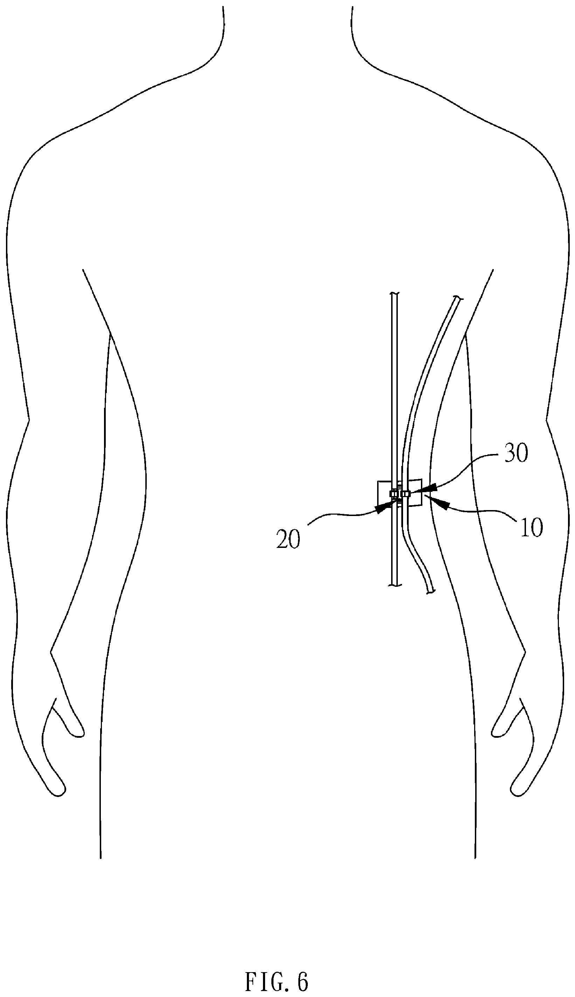

As shown in **FIG. 1** through **FIG. 14,** the present invention provides a medical-tube affixing device for a patient to use. The medical-tube affixing device comprises: an adhesive member **10,** a base **20,** and a clipping assembly **30.** The adhesive member **10** has its one side adhering to the patient. The base **20** is U-shaped base and fixed to a reverse side of the adhesive member **10.** The base **20** defines a slide space **21** in the form of a dovetail mortise. The base **20** has two slender, raised retaining portions **22** and two first pivot holes **23.** The retaining portions **22** are located at a middle portion of a bottom of the slide space **21,** and the first pivot holes **23** are located at two diagonally opposite free corners of the base **20** remote from the retaining portions **22.** The clipping assembly **30** includes a first movable fastener **31,** a first pivot **32,** a first slider **33,** a second slider **34,** a second pivot **35,** and a second movable fastener **36.** The first movable fastener **31** is pivotably installed in the slide space **21** at the first pivot holes **23.** The first movable fastener **31** is U-shaped and faces the slide space **21** with its concave surface. The first movable fastener **31** has an outward-facing, convex surface provided with a plurality of transversely extending first external teeth **311** near one end of the first movable fastener **31.** The first movable fastener **31** further has a first through hole **312** passing through its end remote from the first external teeth **311.** The first through hole **312** is locationally corresponding to the first pivot holes **23** in the slide space **21.** The first pivot **32** is pivotably received in the first pivot holes **23** of the base **20** and the first through hole **312** of the first movable fastener **31,** so that the first movable fastener **31** is swingably mounted on the base **20.** The first slider **33** is shaped as a dovetail tenon matching the dovetail-mortise-shaped slide space **21** of the base **20.** The first slider **33** is provided with a flexible, C-shaped first curved arm **331, a** flexible, C-shaped second curved arm **332,** a channel **333** formed as a through hole with a trapezoid cross-section, and a bottom **334.** The first curved arm **331** and the second curved arm **332** are located at a middle portion of a free side of the first slider **33.** The first curved arm **331** and the second curved arm **332** have their concave, inner surfaces facing the first slider **33.** The inner surfaces of the first curved arm **331** and the second curved arm **332** are each formed with a plurality of transversely extending first internal teeth **3311** and second internal teeth **3321.** The first internal teeth **3311** of the first curved arm **331** are configured to engage with the first external teeth **311** of the first movable fastener **31.** The first slider **33** is slidably fit in the slide space **21** of the base **20** and has its bottom **334** retained by the two retaining portions **22** of the base **20** so that it can slide out of the slide space **21** of the base **20** to a limited extent without separating from the base **20** completely. The channel **333** has two stopping portions **3331** raised from its inner walls near one end of the channel **333.** The second slider **34** has a trapezoid body and is slidably fit in the channel **333** of the first slider **33.** The second slider **34** has two second pivot holes **341** formed at two free corners of is upper surface. The two second pivot holes **341** are configured to receive a second pivot **35.** The second slider **34** has its bottom provided with two raised stoppers **342** to be retained by the raised stopping portions **3331** in the channel **333** of the first slider **33.** The second movable fastener **36** is pivotably attached to the second slider **34** by the second pivot **35** passing through the two second pivot holes **341** of the second slider **34.** The second movable fastener **36** is U-shaped and has its concave surface facing the slide space **21.** The second movable fastener **36** has its convex, outer side formed with a plurality of transversely extending second external teeth **361** near one end of the second movable fastener **36,** and further has a second through hole **362** passing through its end remote from the second external teeth **361.** The second through hole **362** are configured to align with the two second pivot holes **341** of the second slider **34,** so that the second pivot **35** when passing through the second through holes **362** and the second pivot holes **341** of the second slider **34** can swingably attach the second movable fastener **36** to the second slider **34.** The second internal teeth **3321** of the second curved arm **332** are configured to engage with the second external teeth **361** of the second movable fastener **36.**

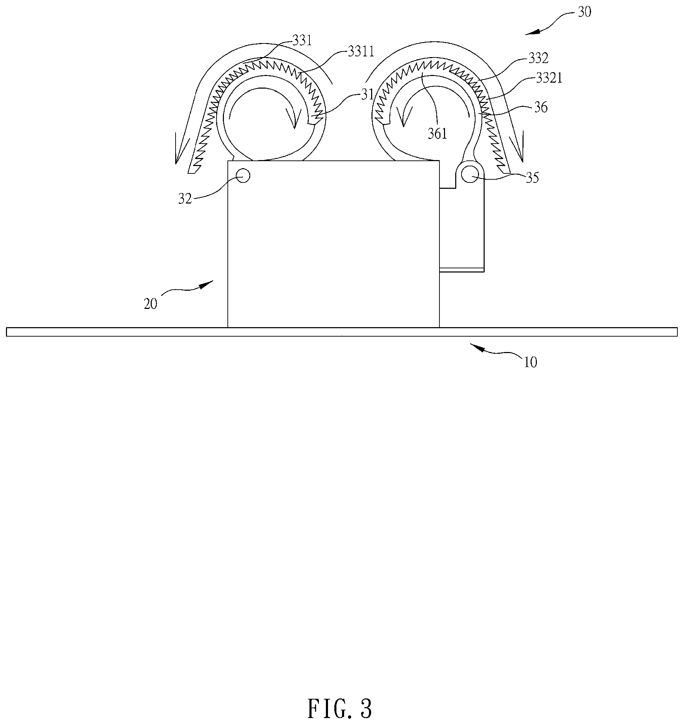

Please refer to **FIG. 3** through **FIG. 6** for operation of the disclosed device. In use, the adhesive side of the adhesive member **10** can be adhesively attached to the patient. Then a medical tube A installed on the body of a patient can be placed at an opening between the first internal teeth **3311** of the first curved arm **331** and the first external teeth **311** of the first movable fastener **31** that are now separated from each other for tube installation. Then the first internal teeth **3311** of the first curved arm **331** and the first external teeth **311** of the first movable fastener **31** can be drawn together to engage with each other. The first slider **33** can slide in the slide space **21** of the base **20** toward the first movable fastener **31.** The first internal teeth **3311** of the first curved arm **331** and the first external teeth **311** of the first movable fastener **31** when engaging with each other jointly define an enclosure for receiving the medical tube **A.** The first internal teeth **3311** of the first curved arm **331** and the first external teeth **311** of the first movable fastener **31** after engaging with each other can be further adjusted to fittingly receive the medical tube **A,** thereby facilitating operation and improving working efficiency. If there is another medical tube **B** installed on the body of the patient, the medical tube **B** may be placed between the second internal teeth **3321** of the second curved arm **332** and the second external teeth **361** of the second movable fastener **36,** so that the second internal teeth **3321** of the second curved arm **332** work with the second external teeth **361** of the second movable fastener **36** to hold the medical tube **B.** The second internal teeth **3321** of the second curved arm **332** are configured to engage with the second external teeth **361** of the second movable fastener **36,** and the second slider **34** can slide in the channel **333** of the first slider **33** toward the second curved arm **332.** The second internal teeth **3321** of the second curved arm **332** and the second external teeth **361** of the second movable fastener **36** when engaging with each other jointly define another adjustable enclosure for fittingly receiving the medical tube **B,** thereby facilitating operation and improving working efficiency. With the configuration and operation described previously, a healthcare worker can efficiently affix multiple medical tubes (such as A and B) to a patient and hold the tubes in position with the convenience in adjusting the clipping assembly **30** to fit the sizes of the used tubes and repositioning the base **20.**

Removal of the medical tube **A** from the disclosed device can be easily achieved by pulling the first curved arm **331** and the first movable fastener **31** apart to separate the first internal teeth **3311** and the first external teeth **311,** and sliding the first slider **33** into the slide space **21** of the base **20.** Similarly, removal of the medical tube **B** can be easily achieved by pulling the second curved arm **332** and the second movable fastener 36 apart to separate the second internal teeth **3321** and the second external teeth **361,** and sliding the second slider **34** out of the channel **333** of the first slider **33.** With use of the disclosed medical-tube affixing device, medical tubes can be easily and firmly attached to/detached from and positioned and/or repositioned with respect to a patient, thereby improving working efficiency of healthcare workers.

With the configuration described previously, the embodiment of the present invention has the following benefits. The disclosed medical-tube affixing device enables a healthcare worker to affix multiple medical tubes to a patient with the adhesive member **10** easily and firmly.

In use of the disclosed medical-tube affixing device, the adhesive side of the adhesive member **10** can be adhesively attached to the patient. Then a medical tube **A** installed on the body of a patient can be placed at an opening between the first internal teeth **3311** of the first curved arm **331** and the first external teeth **311** of the first movable fastener **31** that are now separated from each other for tube installation. Then the first internal teeth **3311** of the first curved arm **331** and the first external teeth **311** of the first movable fastener **31** can be drawn together to engage with each other. The first slider **33** can slide in the slide space **21** of the base **20** toward the first movable fastener **31.** The first internal teeth **3311** of the first curved arm **331** and the first external teeth **311** of the first movable fastener **31** when engaging with each other jointly define an enclosure for receiving the medical tube **A.** The first internal teeth **3311** of the first curved arm **331** and the first external teeth **311** of the first movable fastener **31** after engaging with each other can be further adjusted to fittingly receive the medical tube **A,** thereby facilitating operation and improving working efficiency. If there is another medical tube **B** installed on the body of the patient, the medical tube **B** may be placed between the second internal teeth **3321** of the second curved arm **332** and the second external teeth **361** of the second movable fastener **36,** so that the second internal teeth **3321** of the second curved arm **332** work with the second external teeth **361** of the second movable fastener **36** to hold the medical tube **B.** The second internal teeth **3321** of the second curved arm **332** are configured to engage with the second external teeth **361** of the second movable fastener **36,** and the second slider **34** can slide in the channel **333** of the first slider **33** toward the second curved arm **332.** The second internal teeth **3321** of the second curved arm **332** and the second external teeth **361** of the second movable fastener **36** when engaging with each other jointly define another adjustable enclosure for fittingly receiving the medical tube **B,** thereby facilitating operation and improving working efficiency. With the configuration and operation described previously, a healthcare worker can efficiently affix multiple medical tubes (such as A and B) to a patient and hold the tubes in position with the convenience in adjusting the clipping assembly **30** to fit the sizes of the used tubes and repositioning the base **20.**

Removal of the medical tube **A** from the disclosed device can be easily achieved by pulling the first curved arm **331** and the first movable fastener **31** apart to separate the first internal teeth **3311** and the first external teeth **311,** and sliding the first slider **33** into the slide space **21** of the base **20.** Similarly, removal of the medical tube **B** can be easily achieved by pulling the second curved arm **332** and the second movable fastener **36** apart to separate the second internal teeth **3321** and the second external teeth **361,** and sliding the second slider **34** out of the channel **333** of the first slider **33.** With use of the disclosed medical-tube affixing device, medical tubes can be easily and firmly attached to/detached from and positioned and/or repositioned with respect to a patient, thereby improving working efficiency of healthcare workers.

## Claims

1. A medical-tube affixing device, comprising an adhesive member (10) to adhere to a patient and thereby affixing medical tubes to the patient, wherein the medical-tube affixing device further comprises:
a base (20), being fixed to the adhesive member (10) and defining therein a slide space (21) and two first pivot holes (23), wherein the first pivot holes (23) are formed at two free corners of the base (20); and
a clipping assembly (30), including a first movable fastener (31), a first pivot (32), a first slider (33), a second slider (34), a second pivot (35), and a second movable fastener (36), the first movable fastener (31) being pivotably installed in the slide space (21) at the first pivot holes (23), the first movable fastener (31) facing the slide space (21), the first movable fastener (31) having an outer surface formed with a plurality of transversely extending first external teeth (311) and being formed with a first through hole (312) passing through its end remote from the first external teeth (311), the first through hole (312) being locationally corresponding to the first pivot holes (23) at the slide space (21), the first pivot (32) passing through one of the first pivot holes (23), the first through hole (312) of the first movable fastener (31), and the other of the first pivot holes (23) to swingably attach the first movable fastener (31) to the base (20);
the first slider (33) being shaped to match the slide space (21) of the base (20), the first slider (33) being provided with a first curved arm (331), a second curved arm (332), a channel (333), and a bottom (334), the first curved arm (331) and the second curved arm (332) being located at a middle portion of a free side of the first slider (33), the first curved arm (331) and the second curved arm (332) extending and facing the first slider (33), the first curved arm (331) and the second curved arm (332) having inner surfaces thereof provided with a plurality of transversely extending first internal teeth (3311) and a plurality of second internal teeth (3321), the first internal teeth (3311) of the first curved arm (331) and the first external teeth (311) of the first movable fastener (31) being configured to engage with each other, the first slider (33) being slidably received in the slide space (21) of the base (20) and having the bottom (334) retained by two retaining portions (22) of the base (20) so the first slider (33) is allowed to slide out of the slide space (21) of the base (20) to a limited extent;
the second slider (34), being slidably received in the channel (333) of the first slider (33) and having two second pivot holes (341) formed at two free corners of an upper surface thereof, the two second pivot holes (341) being configured to receive the second pivot (35);
the second movable fastener (36), being pivotably installed at the two second pivot holes (341) of the second slider (34), the second movable fastener (36) being U-shaped and facing the slide space (21), the second movable fastener (36) having an outer surface formed with a plurality of transversely extending second external teeth (361), and having a second through hole (362) passing through an end thereof remote from the second external teeth (361), the second through hole (362) being aligned with the two second pivot holes (341) of the second slider (34), the second pivot (35) passing through one of the second pivot holes (341) of the second slider (34), the second through hole (362), and the other of the second pivot holes (341) of the second slider (34) to swingably attach the second movable fastener (36) to the second slider (34), the second internal teeth (3321) of the second curved arm (332) and the second external teeth (361) of the second movable fastener (36) being configured to engage with each other.

2. The medical-tube affixing device of claim 1, being **characterized in that** the adhesive member (10) adheres to the patient at one side thereof, and the base (20) has a U-shaped body that is fixed to a reverse side of the adhesive member (10).

3. The medical-tube affixing device of claim 1, being **characterized in that** the slide space (21) of the base (20) is shaped as a dovetail mortise, and the two retaining portions (22) that are slender and raised, and the two first pivot holes (23), wherein the two retaining portions (22) are located at a bottom of the slide space (21), and the first pivot holes (23) are located at two diagonally opposite free corners of the base (20) remote from the retaining portions (22), in which the first slider (33) is shaped as a dovetail tenon matching the dovetail-mortise-shaped slide space (21) of the base (20).

4. The medical-tube affixing device of claim 1, being **characterized in that** the first movable fastener (31) is U-shaped.

5. The medical-tube affixing device of claim 1, being **characterized in that** the first curved arm (331) and the second curved arm (332) each have a flexible, C-shaped body, and the channel (333) is formed as a through hole with a trapezoid cross-section.

6. The medical-tube affixing device of claim 1, being **characterized in that** the second slider (34) has a trapezoid body.

7. The medical-tube affixing device of claim 1, being **characterized in that** the channel (333) of the first slider (33) has two stopping portions (3331) raised from inner walls of the channel (333) and near one end of the channel (333), wherein the stopping portions (3331) are configured to engage with two stoppers (342) raised from a bottom of the second slider (34).

## Patentansprüche

1. Ein Befestigungsgerät für medizinische Schläuche, umfassend ein Klebeelement (10), das zum Anhaften an einem Patienten dient und dadurch medizinische Schläuche am Patienten befestigt werden, wobei das Befestigungsgerät für medizinische Schläuche ferner Folgendes umfasst:
eine Basis (20), die an dem Klebeelement (10) befestigt ist und darin einen Gleitraum (21) sowie zwei erste Drehachsenlöcher (23) definiert, wobei die ersten Drehachsenlöcher (23) an zwei freien Ecken der Basis (20) ausgebildet sind; und
eine Klemmanordnung (30), die Folgendes umfasst: ein erstes bewegliches Befestigungselement (31), eine erste Drehachse (32), ein erstes Gleitelement (33), ein zweites Gleitelement (34), eine zweite Drehachse (35) und ein zweites bewegliches Befestigungselement (36), wobei das erste bewegliche Befestigungselement (31) schwenkbar im Gleitraum (21) an den ersten Drehachsenlöchern (23) montiert ist, wobei das erste bewegliche Befestigungselement (31) zum Gleitraum (21) zeigt, wobei das erste bewegliche Befestigungselement (31) eine mit einer Vielzahl quer verlaufender erster Außenzähne (311) versehene Außenfläche aufweist und mit einem ersten Durchgangsloch (312) versehen ist, das durch sein von den ersten Außenzähnen (311) entferntes Ende verläuft, wobei das Durchgangsloch (312) lagemäßig zu den ersten Drehachsenlöchern (23) im Gleitraum (21) korrespondiert, wobei die erste Drehachse (32) durch eines der ersten Drehachsenlöcher (23), das erste Durchgangsloch (312) des ersten beweglichen Befestigungselements (31) und das andere erste Drehachsenloch (23) durchgeführt ist, um das erste bewegliche Befestigungselement (31) schwenkbar an der Basis (20) zu befestigen;
das erste Gleitelement (33), das in einer zum Gleitraum (21) der Basis (20) passenden Form ausgebildet ist, wobei das erste Gleitelement (33) einen ersten gebogenen Arm (331), einen zweiten gebogenen Arm (332), einen Kanal (333) und einen Boden (334) aufweist, wobei sich der erste gebogene Arm (331) und der zweite gebogene Arm (332) an einem mittleren Abschnitt einer freien Seite des ersten Gleitelements (33) befinden, wobei sich der erste gebogene Arm (331) und der zweite gebogene Arm (332) in Richtung des ersten Gleitelements (33) erstrecken und ihm zugewandt sind, wobei die Innenflächen des ersten gebogenen Arms (331) und des zweiten gebogenen Arms (332) mit einer Vielzahl quer verlaufender erster Innenzähne (3311) und zweiter Innenzähne (3321) versehen sind, wobei die ersten Innenzähne (3311) des ersten gebogenen Arms (331) und die ersten Außenzähne (311) des ersten beweglichen Befestigungselements (31) so ausgelegt sind, dass sie miteinander in Eingriff gebracht werden können, wobei das erste Gleitelement (33) verschiebbar im Gleitraum (21) der Basis (20) aufgenommen ist und einen Boden (334) hat, der von zwei Halteabschnitten (22) der Basis (20) gehalten wird, sodass sich das erste Gleitelement (33) nur begrenzt aus dem Gleitraum (21) der Basis (20) herausbewegen lässt;
das zweite Gleitelement (34), das verschiebbar im Kanal (333) des ersten Gleitelements (33) aufgenommen ist und zwei zweite Drehachsenlöcher (341) an zwei freien Ecken seiner oberen Fläche aufweist, wobei die zweiten Drehachsenlöcher (341) zur Aufnahme der zweiten Drehachse (35) dienen;
das zweite bewegliche Befestigungselement (36), das schwenkbar an den beiden zweiten Drehachsenlöchern (341) des zweiten Gleitelements (34) montiert ist, wobei das zweite bewegliche Befestigungselement (36) U-förmig ausgebildet und dem Gleitraum (21) zugewandt ist, wobei das zweite bewegliche Befestigungselement (36) eine mit einer Vielzahl quer verlaufender zweiter Außenzähne (361) versehene Außenfläche aufweist und mit einem zweiten Durchgangsloch (362) versehen ist, das durch sein von den zweiten Außenzähnen (361) entferntes Ende verläuft, wobei das zweite Durchgangsloch (362) mit den zweiten Drehachsenlöchern (341) des zweiten Gleitelements (34) ausgerichtet ist, wobei die zweite Drehachse (35) durch eines der zweiten Drehachsenlöcher (341) des zweiten Gleitelements (34), das zweite Durchgangsloch (362) und das andere zweite Drehachsenloch (341) des zweiten Gleitelements (34) durchgeführt ist, um das zweite bewegliche Befestigungselement (36) schwenkbar am zweiten Gleitelement (34) zu befestigen, wobei die zweiten Innenzähne (3321) des zweiten gebogenen Arms (332) und die zweiten Außenzähne (361) des zweiten beweglichen Befestigungselements (36) so ausgelegt sind, dass sie miteinander in Eingriff gebracht werden können.

2. Befestigungsgerät für medizinische Schläuche nach Anspruch 1, **dadurch gekennzeichnet, dass** das Klebeelement (10) an seiner einen Seite am Patienten haftet und die Basis (20) einen U-förmigen Körper aufweist, der an der Rückseite des Klebeelements (10) befestigt ist.

3. Befestigungsgerät für medizinische Schläuche nach Anspruch 1, **dadurch gekennzeichnet, dass** der Gleitraum (21) der Basis (20) in Form einer Schwalbenschwanznut ausgebildet ist und die Basis zwei schlanke, erhabene Halteabschnitte (22) und zwei erste Drehachsenlöcher (23) aufweist, wobei sich die beiden Halteabschnitte (22) am Boden des Gleitraums (21) und die ersten Drehachsenlöcher (23) an zwei diagonal gegenüberliegenden, von den Halteabschnitten (22) entfernten freien Ecken der Basis (20) befinden, worin ein erstes Gleitelement (33) in Form eines Schwalbenschwanzzapfens gestaltet ist, der zum schwalbenschwanznutförmigen Gleitraum (21) der Basis (20) passt.

4. Befestigungsgerät für medizinische Schläuche nach Anspruch 1, **dadurch gekennzeichnet, dass** das erste bewegliche Befestigungselement (31) U-förmig ausgebildet ist.

5. Befestigungsgerät für medizinische Schläuche nach Anspruch 1, **dadurch gekennzeichnet, dass** der erste gebogene Arm (331) und der zweite gebogene Arm (332) jeweils einen flexiblen, C-förmigen Körper haben, und der Kanal (333) als Durchgangsloch mit einem trapezförmigen Querschnitt ausgebildet ist.

6. Befestigungsgerät für medizinische Schläuche nach Anspruch 1, **dadurch gekennzeichnet, dass** das zweite Gleitelement (34) einen trapezförmigen Körper aufweist.

7. Befestigungsgerät für medizinische Schläuche nach Anspruch 1, **dadurch gekennzeichnet, dass** der Kanal (333) des ersten Gleitelements (33) zwei Anschlagabschnitte (3331) aufweist, die sich von den Innenwänden des Kanals (333) nahe einem Ende desselben erheben, wobei die Anschlagabschnitte (3331) so konfiguriert sind, dass sie mit zwei von der Unterseite des zweiten Gleitelements (34) erhabenen Anschlägen (342) in Eingriff stehen.

## Revendications

1. Dispositif de fixation de tubes médicaux, comprenant un élément adhésif (10) pour adhérer à un patient et ainsi fixer des tubes médicaux au patient, le dispositif de fixation de tubes médicaux comprenant en outre :
une base (20), fixée à l'élément adhésif (10) et définissant dans celle-ci un espace de coulissement (21) et deux premiers trous de pivot (23), les premiers trous de pivot (23) étant formés à deux coins libres de la base (20) ; et
un ensemble de clippage (30), comprenant une première attache mobile (31), un premier pivot (32), un premier coulisseau (33), un second coulisseau (34), un second pivot (35) et une seconde attache mobile (36), la première attache mobile (31) étant installée de manière pivotante dans l'espace de coulissement (21) aux premiers trous de pivot (23), la première attache mobile (31) faisant face à l'espace de coulissement (21), la première attache mobile (31) ayant une surface externe formée avec une pluralité de premières dents externes (311) s'étendant transversalement et étant formée avec un premier trou traversant (312) passant à travers son extrémité éloignée des premières dents externes (311), le premier trou traversant (312) correspondant localement aux premiers trous de pivot (23) à l'espace de coulissement (21), le premier pivot (32) passant à travers l'un des premiers trous de pivot (23), le premier trou traversant (312) de la première attache mobile (31) et l'autre des premiers trous de pivot (23) pour attacher de manière oscillante la première attache mobile (31) à la base (20) ;
le premier coulisseau (33) étant façonné pour correspondre à l'espace de coulissement (21) de la base (20), le premier coulisseau (33) comportant un premier bras incurvé (331), un second bras incurvé (332), un canal (333) et un fond (334), le premier bras incurvé (331) et le second bras incurvé (332) étant situés à une partie moyenne d'un côté libre du premier coulisseau (33), le premier bras incurvé (331) et le second bras incurvé (332) s'étendant et faisant face au premier coulisseau (33), le premier bras incurvé (331) et le second bras incurvé (332) ayant des surfaces internes de ceux-ci comportant une pluralité de premières dents internes (3311) s'étendant transversalement et une pluralité de secondes dents internes (3321), les premières dents internes (3311) du premier bras incurvé (331) et les premières dents externes (311) de la première attache mobile (31) étant configurées pour s'engager les unes avec les autres, le premier coulisseau (33) étant reçu de manière coulissante dans l'espace de coulissement (21) de la base (20) et ayant le fond (334) retenu par deux parties de retenue (22) de la base (20) de telle sorte que le premier coulisseau (33) est autorisé à coulisser hors de l'espace de coulissement (21) de la base (20) dans une mesure limitée ;
le second coulisseau (34) étant reçu de manière coulissante dans le canal (333) du premier coulisseau (33) et ayant deux seconds trous de pivot (341) formés à deux coins libres d'une surface supérieure de celui-ci, les deux seconds trous de pivot (341) étant configurés pour recevoir le second pivot (35) ;
la seconde attache mobile (36) étant installée de manière pivotante aux deux seconds trous de pivot (341) du second coulisseau (34), la seconde attache mobile (36) étant en forme de U et faisant face à l'espace de coulissement (21), la seconde attache mobile (36) ayant une surface externe formée avec une pluralité de secondes dents externes (361) s'étendant transversalement, et ayant un second trou traversant (362) passant à travers une extrémité de celle-ci éloignée des secondes dents externes (361), le second trou traversant (362) étant aligné avec les deux seconds trous de pivot (341) du second coulisseau (34), le second pivot (35) passant à travers l'un des seconds trous de pivot (341) du second coulisseau (34), le second trou traversant (362) et l'autre des seconds trous de pivot (341) du second coulisseau (34) pour attacher de manière oscillante la seconde attache mobile (36) au second coulisseau (34), les secondes dents internes (3321) du second bras incurvé (332) et les secondes dents externes (361) de la seconde attache mobile (36) étant configurées pour s'engager les unes avec les autres.

2. Dispositif de fixation de tubes médicaux selon la revendication 1, **caractérisé par le fait que** l'élément adhésif (10) adhère au patient d'un côté de celui-ci, et la base (20) a un corps en forme de U qui est fixé à un côté opposé de l'élément adhésif (10).

3. Dispositif de fixation de tubes médicaux selon la revendication 1, **caractérisé par le fait que** l'espace de coulissement (21) de la base (20) est façonné en tant que mortaise à queue d'aronde, et les deux parties de retenue (22) qui sont plus minces et relevées, et les deux premiers trous de pivot (23), les deux parties de retenue (22) étant situées à un fond de l'espace de coulissement (21), et les premiers trous de pivot (23) étant situés à deux coins libres diagonalement opposés de la base (20), à distance des parties de retenue (22), le premier coulisseau (33) ayant la forme d'un tenon à queue d'aronde correspondant à l'espace de coulissement (21) en forme de mortaise à queue d'aronde de la base (20).

4. Dispositif de fixation de tubes médicaux selon la revendication 1, **caractérisé par le fait que** la première attache mobile (31) est en forme de U.

5. Dispositif de fixation de tubes médicaux selon la revendication 1, **caractérisé par le fait que** le premier bras incurvé (331) et le second bras incurvé (332) ont chacun un corps en forme de C, flexible, et le canal (333) est formé en tant que trou traversant avec une section trapézoïdale.

6. Dispositif de fixation de tubes médicaux selon la revendication 1, **caractérisé par le fait que** le second coulisseau (34) a un corps trapézoïdal.

7. Dispositif de fixation de tubes médicaux selon la revendication 1, **caractérisé par le fait que** le canal (333) du premier coulisseau (33) a deux parties d'arrêt (3331) relevées à partir des parois internes du canal (333) et près d'une extrémité du canal (333), les parties d'arrêt (3331) étant configurées pour s'engager avec deux butées (342) relevées à partir d'un fond du second coulisseau (34).
